# EUROPEAN PATENT APPLICATION

(11) **EP 4 825 370 A1**
(43) Date of publication of application: **21.10.2026**
(21) Application number: 24901786.4
(22) Date of filing: 16.12.2024
(51) Int. Cl.: A61K 8/64, A61Q 19/00, A61K 38/07, A61P 17/04, A61P 17/08

(54) **COSMETIC, COSMECEUTICAL AND/OR PHARMACEUTICAL COMPOSITION, USES AND METHODS**

(30) Priority: 15.12.2023 BR 102023026522
(71) Applicant: Chemyunion Ltda, 18087-800 Sorocaba (BR)
(72) Inventor: KATEKAWA, Edson, Sorocaba (BR); ROGERIO PRINCIVAL, Cleverson, Sorocaba (BR); MUSSI, Lilian, Sorocaba (BR); VIDAL MAGALHÃES, Wagner, Sorocaba (BR)
(74) Representative: Hoffmann Eitle
(86) International application number: PCT/BR2024/050587
(87) International publication number: WO 2025/123114

(57) **Abstract**

The present invention describes new cosmetic, cosmeceutical and/or pharmaceutical compositions, as well as their uses and methods useful for alleviating conditions resulting from inflammatory processes, sensitive skin, rosacea, atopic dermatitis, eczema; and/or; for alleviating the sensation of discomfort and pain; modulation of the CB2 receptor; and/or; for providing benefits on problems relating to skin hyperchromias and disorders relating to increased sebogenesis.

## Description

### Technical Field of the Invention

The present invention describes new cosmetic, cosmeceutical and/or pharmaceutical compositions, as well as their uses and methods useful for alleviating conditions resulting from inflammatory processes, sensitive skin, rosacea, atopic dermatitis, eczema; modulation of the CB2 receptor; and/or; for alleviating the sensation of discomfort and pain; and/or; for providing benefits on problems relating to skin hyperchromias and disorders relating to increased sebogenesis.

### Background of the Invention

Plants of the genus Cannabis have a long history of use by humans, with evidence that they were used more than 10,000 years ago for improvement of ceramics and may have been one of the first known agricultural crops (TEXTILE WORLD, 2020).

Cannabis has been used for food, production of paper and textiles and, more recently, as a raw material for biodegradable plastics. Among the secondary metabolites found in these plants are tetrahydrocannabinol (THC), cannabidiol (CBD), cannabinol (CBN), cannabigerol (CBG), tetrahydrocannabivarin (THCV) and other substances classified as cannabinoids. As with the secondary metabolites of other botanical species, cannabinoids have been studied for the purpose of finding their bioactive properties in the human body.

The use of medicinal Cannabis may have begun around 2,000 BC, although the compendium documenting it is from the Han dynasty (221 BC - 220 AD) (CROCQ, 2020). It should be noted that not all cannabinoids have psychoactive effects, THC being the main psychoactive present in Cannabis.

The hypothalamic-pituitary-adrenal (HPA) axis is a neuroendocrine system that regulates various bodily processes through the release of hormones. The skin not only expresses proteins of this axis, but also responds to its signaling, suggesting the existence of a brain-skin axis. Thus, it is understood that substances having biological activity in the brain may eventually trigger biological processes in the skin. Moreover, imbalance of the brain-skin axis has been linked to inflammatory dermatological disorders, such as psoriasis (MOON, 2013).

The endocannabinoid system (ECS) is a neuromodulatory system composed of cannabinoid receptors, endogenous cannabinoid substances (endocannabinoids) and a set of enzymes responsible for the synthesis and degradation of endocannabinoids. The ECS acts in the central nervous system (including the brain) and in the peripheral nervous system. Among the cannabinoid receptors, cannabinoid receptor 1 (CB1) stands out due to its abundance, although other receptors are also modulated by cannabinoid substances - such as cannabinoid receptor 2 (CB2), transient receptor potential channels (TRPs) and peroxisome proliferator-activated receptors (PPARs), for example. There is evidence that the main cannabinoid receptors, CB1 and CB2, are also expressed in the skin, suggesting that the ECS may integrate the brain-skin axis (THE PROTEIN ATLAS).

Despite proof of CBD efficacy in various areas, such as for the treatment of epilepsy and the great potential for treatment of other disorders such as migraine (GRECO, 2023) and dementia (ALEXANDRI, 2023), the possible presence of the psychoactive substance tetrahydrocannabinol (THC) in Cannabis extracts (PINO, 2023) causes products based on these plants to be considered, at a minimum, controversial - for example, intoxication by THC through the use of Cannabis oil marketed as CBD oil has already been reported (MOUSSA, 2023).

In addition to the toxicological aspect, which could be overcome by conducting chemical analyses for evaluation and control of narcotic substances in CBD oil, there are other elements that impair the full dissemination of this ingredient in the market, such as regulatory, legal and supply chain issues.

In Brazil, for example, although the sale of CBD-based medicines has been authorized by the National Health Surveillance Agency (Anvisa), local production of CBD (at least, from Cannabis) remains prohibited. Such restrictions make CBD scarcely accessible, both from the economic side and from a practical point of view.

In the cosmetic field, the first consumer products containing CBD reached the market around 2018, where the substance was used mainly as an anti-inflammatory agent for the skin. In 2020, almost 300 new products containing CBD were launched - and, since then, there has been a downward trend in this number. This may be explained, in part, by the controversial character of this ingredient.

In the search for the prior art, the following patent documents were detected.

WO2008014414 published on January 31, 2008 describes methods for attenuating the release of inflammatory mediators using the MANS peptide (MA-GAQFSKTAAKGEAAAERPGEAAVA - SEQ ID NO. 1) and derivatives, wherein "MA" is a myristoyl group coupled to the N-terminal portion. With regard to the published experimental data, it is not possible to state that any terminal portion of the MANS peptide has inhibitory activity of inflammatory mediators. That is, the peptides MA-SKTAAKGEAAAERPGEAAVA (SEQ ID NO. 15) and MA-RPGEAAVA (SEQ ID NO. 153) did not show any inhibition of EPO release, and the peptide Ac-RPGEAAVA did not show any inhibition in lysozyme release. The peptide closest to the present invention tested was RPGEAAVA, with only 50% identity relative to the peptide tested by the present invention, but with a totally distinct structure due to the number of amino acids present (8 vs 4). Additionally, there are no experimental data indicating action on IL-6, IFN-gamma and KC levels (analog of IL-8 in murine) for peptides containing tetrapeptide sequences. The tests indicated with the MANS peptide (with only 17% identity to the tetrapeptides claimed herein) are totally abstract and no factual experimental results are presented. Also highlighted are results obtained with other sequences that showed results similar to SEQ ID NO. 153 (50% identity), as well as SEQ ID. 106. None of them has identity with the peptides tested by the present patent application. If any effect is observed it would be with SEQ ID NO. 106, which does not contain "AAVA". Therefore, it is evident that, with respect to the information provided in WO2008014414, the AAVA sequence could not be inferred as being responsible for the effects. Thus, it is possible to conclude that the technical information brought in the present patent application under analysis demonstrates surprising technical effects found.

WO2023212443 published on November 2, 2023 describes methods of treating a disease or condition that produces inflammation and/or itching in the skin. Although the document generically indicates that it uses peptide fragments of the protein entitled by the document as MARCKS (GAQFSKTAAKGEAAAERPGEAAVA), the only factual / experimental test is with a peptide of a sequence totally distinct from that claimed in the present patent application and of sequence (Ac-GAQFSKTAAK-OH). Thus, no tetrapeptide or similar has support or is sufficiently described so that it may be relevant to the present patent application.

Therefore, since various effective therapeutic applications of cannabinoids have already been demonstrated, the search for substances that act on the endocannabinoid system safely and effectively as an alternative to those found naturally in plants of the genus Cannabis may be considered important.

### Summary of the Invention

In this regard, the patent application presents new cosmetic, cosmeceutical and/or pharmaceutical peptide compositions containing at least one peptide of general formula:

R1-A1-A2-A3-A4-R2

wherein:
A1 is an amino acid containing an apolar side chain and selected from the group Ala, Ile, Leu, Val, Gly, Pro, Ile, Gly, norleucine, norvaline, 2-aminobutyric acid and sarcosine;
A2 is an amino acid containing an apolar side chain and selected from the group Ala, Ile, Leu, Val, Gly, Pro, Ile, Gly, norleucine, norvaline, 2-aminobutyric acid and sarcosine;
A3 is an amino acid containing an apolar side chain and selected from the group Ala, Ile, Leu, Val, Gly, Pro, Ile, Gly, norleucine, norvaline, 2-aminobutyric acid and sarcosine;
A4 is an amino acid containing an apolar side chain and selected from the group Ala, Ile, Leu, Val Gly, Pro, Ile, Gly-, norleucine, norvaline, 2-aminobutyric acid and sarcosine.
R1 is an N-terminal chain modifier selected from the group consisting of H, substituted or unsubstituted acyclic aliphatic, substituted or unsubstituted cyclic aliphatic, substituted or unsubstituted heterocyclic, substituted or unsubstituted heteroarylalkyl, substituted or unsubstituted aryl, substituted or unsubstituted arylalkyl, substituted or unsubstituted acyl, substituted or unsubstituted carbonyl;
R2 is selected from the group consisting of -NR3R4, OR3 and -SR3, wherein R3 and R4 are independently selected from the group consisting of H, substituted or unsubstituted acyclic aliphatic, substituted or unsubstituted cyclic aliphatic, substituted or unsubstituted heterocyclic, substituted or unsubstituted heteroarylalkyl, substituted or unsubstituted aryl, substituted or unsubstituted arylalkyl, substituted or unsubstituted acyl, substituted or unsubstituted carbonyl, as well as their stereoisomers, mixture and/or their cosmetically or pharmaceutically acceptable salts.

Said peptides are particularly useful for acting on the endocannabinoid system (more specifically, for presenting high affinity for the CB2 receptor; and concomitant low or no affinity for the CB1 receptor) and, thus, providing the therapeutic benefits of a cannabinoid but without producing psychotropic effects. Such peptide can be produced with very high purity, eliminating adverse effects such as those observed in some oils extracted from Cannabis.

Further, in the studies carried out and that will be demonstrated throughout this patent application, it was observed that the peptide acts as an anti-inflammatory and/or antinociceptive substance, in addition to modulating melanin production, with performance equivalent to or superior to other substances used for the same purposes in the cosmetic field.

The peptides presented in this invention are safe and effective substances in reducing inflammatory conditions, as proven by studies in *in vitro*, *ex vivo* and *in vivo* models.

### Description of the Figures

Figure 1: Effect of the peptide on IL-1α synthesis in human keratinocytes, after 6 hours of treatment in comparative differences among the tested groups. ^{##}P<0.01 relative to the basal control group. **P<0.01 relative to PMA/LPS (Dunnett multiple comparisons test).
Figure 2: Effect of the peptide on IL-6 synthesis in human keratinocytes, after 48 hours of treatment in comparative differences among the tested groups. ^{##}P<0.01 relative to the basal control group. **P<0.01 relative to PMA/LPS (Dunnett multiple comparisons test).
Figure 3: Semi-quantification of melanin levels. Data obtained by image recording of histological sections of human skin fragments previously treated with formulation without active ingredient (Placebo) and treated with the same formulation containing peptide SEQ ID No. 1 (Succinyl-L-alanyl-L-alanyl-L-valyl-L-alanine) (at 200 ppm or 100 ppm). ^{##}P<0.01 relative to the basal control group. **p<0.01 compared to the placebo group.

### Detailed Description of the Invention

The present detailed description of the invention sets forth some nonlimiting definitions of the main terminologies and technical characteristics employed throughout this patent application, as well as provides examples of some of the embodiments of the present invention so that it may be reproduced by a person skilled in the art.

The present invention describes the use of tetrapeptides capable of alleviating conditions resulting from inflammatory processes, such as, but not limited to, sensitive skin, rosacea, atopic dermatitis, eczema, among others. Simultaneously, the present invention is intended for applications of alleviating the sensation of discomfort and pain. Additionally, the invention provides benefits on problems relating to skin hyperchromias and disorders relating to increased sebogenesis.

In a preferred embodiment of the present invention, R1 and R2 described in the general formula defining the group of peptides are not proteinogenic amino acids.

Wherein for the description of amino acids in this document, the 1-letter or 3-letter abbreviations may be used, as widely adopted by the scientific community and by the International Nucleotide Sequence Database.

This invention further includes any and all conservative substitutions of sequence SEQ ID No. 1. Conservative substitution is understood to mean substitution of one amino acid by another having similar hydrophobicity, polarity and/or side chain, so as to produce little or no three-dimensional or functional alteration of the peptide.

Among these peptides are, as examples, Succinyl-Ala-Ala-Val-Ala-OH (SEQ ID No. 1), Pal-Ala-Ala-Val-Ala-OH (SEQ ID No. 2), Ac-Ala-Ala-Val-Ala-OH (SEQ ID No. 3), Succinyl-Gly-Ala-Val-Ala-OH (SEQ ID No. 4) and Ac-Gly-Ala-Val-Ala-OH (SEQ ID No. 5), wherein the prefix Pal- identifies an N-palmitoylated peptide and the prefix Ac- identifies an N-acetylated peptide.

Such peptides may be obtained by various methods, preferably by solid-phase chemical synthesis or in homogeneous systems, without excluding other possibilities, such as biotechnology, hydrolysis and/or purification of proteins and other methods that may exist or come to exist.

These peptides may be used in a concentration range between 0.001% (w/w) and 10.00% (w/w), preferably close to 0.01% (w/w), applied in a cosmetic or pharmaceutical formulation suitable for human or veterinary use.

As a cosmetic or pharmaceutical formulation suitable for human or veterinary use, there is understood any and every type of solvent, dispersion medium, encapsulations, permeation-enhancing agents, surfactants, preservatives and other ingredients, alone or in combination, and that are physiologically compatible.

### Example 1: In vitro evaluation of inhibition of IL-1 alpha production.

Human keratinocytes were seeded in 75 cm² flasks cultured and expanded in a humidified incubator at 37°C in the presence of 5% CO₂, using specific culture medium. Upon reaching 70-80% confluence, the cells were trypsinized and seeded in 6-well plates for subsequent incubation with the test product and evaluation of the proposed parameter. The cells were incubated with 2 non-cytotoxic concentrations for the peptide (120 ppm and 60 ppm). Next, the cells were incubated under the same culture conditions cited above for 6 hours for subsequent collection of the cell lysate. Next, quantification of the pro-inflammatory mediator IL-1alpha was performed by the ELISA technique.

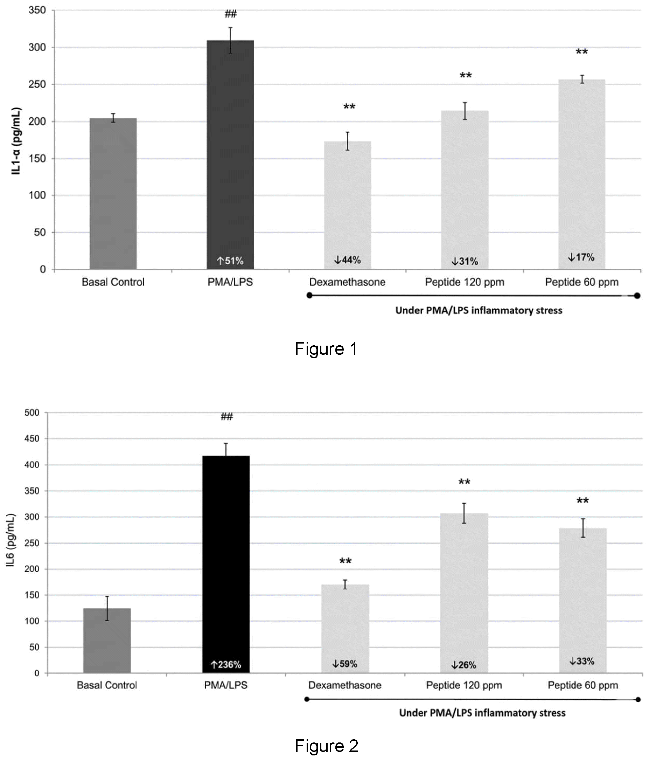

The results expressed in Figure 1 demonstrate the anti-inflammatory capacity of peptide Succinyl-Ala-Ala-Val-Ala-OH (SEQ ID No. 1). At the concentration of 120 ppm, the peptide reduces IL-1α levels by 31% relative to the PMA/LPS-stimulated group.

### Example 2: In vitro evaluation of inhibition of IL-6 production.

Human keratinocytes were seeded in 75 cm² flasks cultured and expanded in a humidified incubator at 37°C in the presence of 5% CO₂, using specific culture medium. Upon reaching 70-80% confluence, the cells were trypsinized and seeded in 6-well plates for subsequent incubation with the test product and evaluation of the proposed parameter. The cells were incubated with 2 non-cytotoxic concentrations for the peptide (120 ppm and 60 ppm). Next, the cells were incubated under the same culture conditions cited above for 48 hours for subsequent collection of the cell supernatant. Next, quantification of the pro-inflammatory mediator IL-6 was performed by the ELISA technique.

The results expressed in Figure 2 demonstrate the anti-inflammatory capacity of peptide Succinyl-Ala-Ala-Val-Ala-OH (SEQ ID No. 1). At the concentration of 60 ppm, the peptide reduces IL-6 levels by 33% relative to the PMA/LPS-stimulated group.

### Example 3: Evaluation of inhibition of melanin synthesis in human skin fragments.

Skin explants, obtained from a surgical procedure performed by an ophthalmologic plastic surgery clinic, were treated for 72 h with formulation containing peptide Succinyl-Ala-Ala-Val-Ala-OH (SEQ ID No. 1) at 200 ppm or 100 ppm and with the same formulation without addition of the peptide (placebo).

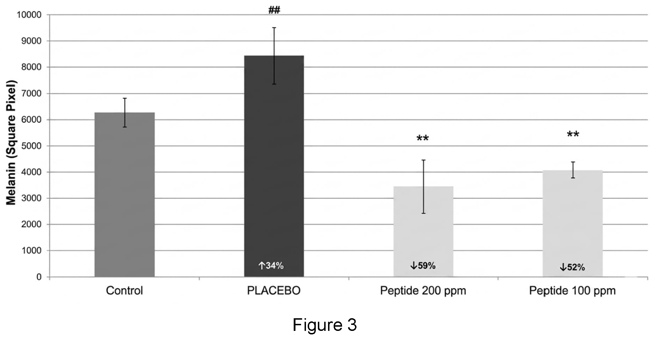

After incubation with the test products, as described above, the skin fragments were fixed in 4% paraformaldehyde (pH 7.4) for 24 hours. After fixation, the material was transferred to 30% Sucrose solution for cryopreservation and then embedded in tissue freezing medium, followed by serial sections of about 10 µm thickness made by cryostat and collected directly on silanized glass slides. Next, the skin sections were stained by the Fontana Masson technique. Subsequently, the slides were analyzed and recorded by an optical microscope coupled to a photographic camera. The results may be verified in Figure 3.

### Example 4: Preparation of a cosmetic composition comprising peptide SEQ ID No. 1.

A cosmetic composition is prepared whose ingredients are presented in Table 1 below. In an appropriate vessel, the Phase B ingredient was added and heated to 75-80°C. Upon reaching the temperature, the Phase A ingredient was added under constant stirring while maintaining the temperature for 10 minutes. Next, cooling was started to 40-45°C under stirring. Subsequently, the Phase C ingredients were added, one by one, under stirring and homogenized for a further 10 minutes, obtaining a cosmetic composition with the proportions shown in Table 1. The composition is a cream suitable for topical use.

**Table 1. Cosmetic composition comprising peptide SEQ ID No. 1.**

| **Phase** | **INCI** | **% (w/w)** | **Function** |
|---|---|---|---|
| A | Cetearyl Alcohol (and) Polysorbate 60 | 12.00 | Emulsifier |
| B | Water | 86.00 | Solvent |
| C | Benzyl Alcohol | 0.90 | Preservative |
| | Ethylhexylglycerin | 0.10 | Preservative |
| | Propanodiol | 0.99 | Humectant |
| | Peptide SEQ ID No. 1 | 0.01 | Active |

### Example 5: In vivo study with the composition of Example 4, testing the efficacy of peptide SEQ ID No. 1 for induced anti-erythema action in volunteers.

In this study, 15 volunteers of both sexes, phototypes II or III, between 20 and 55 years of age were included. In the dorsal region, each volunteer had two 2 cm² quadrants demarcated, and both exposed to UV-B radiation with a solar simulator (ULTRAVITALUX LAMP - OSRAM), at a dose of 4J/cm². Immediately thereafter, formulation was applied in each quadrant (placebo cream - without active ingredient - cream containing the peptide at 100 ppm). Colorimetry measurements to evaluate reduction of erythema (by means of parameter a*), in addition to subjective measurement of the sensation of heat (guided by an opinion survey questionnaire with psychometric responses to identify the level of affirmation of the observed efficacy), were carried out immediately (T0), 30 minutes (T30), 1 h (T60) and 2 h (T120) after application of the formulations. The results for reduction of induced erythema and relief of the sensation of heat are shown in Tables 2 and 3.

The efficacy of the product is evaluated by assessment of the lightening parameters (L, ITA, Melanin Index): L (Luminosity); ITA (Individual Typology Angle); Melanin Index. Table 2 shows the percentage variations relative to the initial time of the parameters related to pigmentation.

**Table 2. Variation of parameter a*.**

| | VARIATION - DELTA a* | | |
|---|---|---|---|
| | T30 -T0 | T60 - T0 | T120 -T0 |
| Peptide Cream | +1.43 | +1.23 | +2.66 |
| Placebo Cream | +1.50 | +1.7 | +4.39 |

**Table 3. Subjective evaluation Immediate relief/cooling sensation.**

| | % Participants who felt a sensation of relief | | |
|---|---|---|---|
| | T30 | T60 | T120 |
| Peptide Cream | 80 | 60 | 100 |
| Placebo Cream | 47 | 33 | 33 |

The products resulting from the use of the teachings of the present invention may be used as human or veterinary cosmetics, cosmeceuticals or drugs, for topical use capable of alleviating conditions resulting from inflammatory processes, such as, but not limited to, sensitive skin, rosacea, atopic dermatitis, eczema, among others. Simultaneously, the present invention is intended for applications of alleviating the sensation of discomfort and pain. Additionally, the invention promotes benefits on problems relating to skin hyperchromias and disorders relating to increased sebogenesis. Included in this list of applications are cosmetics, creams, lotions, serums, ointments, rinse-off products.

The examples disclosed herein are intended only to exemplify some of the innumerable forms of embodiment and use of the present invention, without limiting the interpretation of its scope and breadth, as well as any alternative forms and configurational variations thereof that will be defined from the claims presented herein.

## Claims

1. Cosmetic, cosmeceutical and/or pharmaceutical composition **characterized by** comprising at least one compound comprising the general formula:
R1-A1-A2-A3-A4-R2
wherein:
A1 is an amino acid containing an apolar side chain and selected from the group Ala, Ile, Leu, Val, Gly, Pro, Ile, Gly, norleucine, norvaline, 2-aminobutyric acid and sarcosine;
A2 is an amino acid containing an apolar side chain and selected from the group Ala, Ile, Leu, Val, Gly, Pro, Ile, Gly, norleucine, norvaline, 2-aminobutyric acid and sarcosine;
A3 is an amino acid containing an apolar side chain and selected from the group Ala, Ile, Leu, Val, Gly, Pro, Ile, Gly, norleucine, norvaline, 2-aminobutyric acid and sarcosine;
A4 is an amino acid containing an apolar side chain and selected from the group Ala, Ile, Leu, Val Gly, Pro, Ile, Gly-, norleucine, norvaline, 2-aminobutyric acid and sarcosine; wherein;
R1 is an N-terminal chain modifier selected from the group consisting of H, substituted or unsubstituted acyclic aliphatic, substituted or unsubstituted cyclic aliphatic, substituted or unsubstituted heterocyclic, substituted or unsubstituted heteroarylalkyl, substituted or unsubstituted aryl, substituted or unsubstituted arylalkyl, substituted or unsubstituted acyl, substituted or unsubstituted carbonyl;
R2 is selected from the group consisting of -NR3R4, OR3 and -SR3, wherein R3 and R4 are independently selected from the group consisting of H, substituted or unsubstituted acyclic aliphatic, substituted or unsubstituted cyclic aliphatic, substituted or unsubstituted heterocyclic, substituted or unsubstituted heteroarylalkyl, substituted or unsubstituted aryl, substituted or unsubstituted arylalkyl, substituted or unsubstituted acyl, substituted or unsubstituted carbonyl;
their stereoisomers, mixture and/or their cosmetically or pharmaceutically acceptable salts.

2. Composition, according to claim 1, **characterized in that** the compound is at least one peptide selected from among Succinyl-Ala-Ala-Val-Ala-OH (SEQ ID No. 1), Pal-Ala-Ala-Val-Ala-OH (SEQ ID No. 2), Ac-Ala-Ala-Val-Ala-OH (SEQ ID No. 3), Succinyl-Gly-Ala-Val-Ala-OH (SEQ ID No. 4) and Ac-Gly-Ala-Val-Ala-OH (SEQ ID No. 5), wherein the prefix Pal- identifies an N-palmitoylated peptide and the prefix Ac-identifies an N-acetylated peptide.

3. Composition, according to claim 1 or 2, **characterized in that** it is for inhibition of IL-1 alpha production, inhibition of IL-6 production, inhibition of melanin synthesis in human skin fragments and anti-erythema action; and/or; for alleviating conditions resulting from inflammatory processes, sensitive skin, rosacea, atopic dermatitis, eczema; and/or; for alleviating the sensation of discomfort and pain; and/or; for providing benefits on problems relating to skin hyperchromias and disorders relating to increased sebogenesis.

4. Composition, according to claim 1, 2 or 3, **characterized in that** it is for topical use.

5. Composition, according to any one of the preceding claims, **characterized in that** it is in the form of a cosmetic, cream, lotion, serum, ointment or rinse-off product.

6. Composition, according to any one of the preceding claims, **characterized in that** the peptides are in a concentration range between 0.001% (w/w) and 10.00% (w/w).

7. Composition, according to any one of the preceding claims, **characterized in that** the peptides are in a concentration range between 0.001% (w/w) and 0.1% (w/w).

8. Use of a composition as defined in any one of claims 1 to 7, **characterized in that** it is for producing a cosmetic, cosmeceutical and/or pharmaceutical composition for inhibition of IL-1 alpha production, inhibition of IL-6 production, inhibition of melanin synthesis in human skin fragments and anti-erythema action; and/or; for topical use to alleviate conditions resulting from inflammatory processes, sensitive skin, rosacea, atopic dermatitis, eczema; and/or; for alleviating the sensation of discomfort and pain; modulation of the CB2 receptor and/or; for providing benefits on problems relating to skin hyperchromias and disorders relating to increased sebogenesis in a human or animal.

9. Method for inhibition of IL-1 alpha production, inhibition of IL-6 production, inhibition of melanin synthesis in human skin fragments and anti-erythema action; and/or; for alleviating conditions resulting from inflammatory processes, sensitive skin, rosacea, atopic dermatitis, eczema; and/or; for alleviating the sensation of discomfort and pain; modulation of the CB2 receptor and/or; for providing benefits on problems relating to skin hyperchromias and disorders relating to increased sebogenesis in a human or animal **characterized by** comprising application to a human or animal of a cosmetically, cosmeceutically and/or pharmaceutically effective amount of a composition as defined in any one of claims 1 to 7.
